Europäisches Patentamt

**European Patent Office**

**Office européen des brevets**

(19)

(11) Veröffentlichungsnummer: **0 115 564**
**B1**

(12) **EUROPÄISCHE PATENTSCHRIFT**

(45) Veröffentlichungstag der Patentschrift:
**24.06.87**

(51) Int. Cl.⁴: **A 61 F 2/30**

(21) Anmeldenummer: **83108197.1**

(22) Anmeldetag: **19.08.83**

(54) Metallene Einsteckhülse für den Verankerungsschaft einer Kleingelenk-Prothese.

(30) Priorität: **16.12.82  CH 7326/82**

(43) Veröffentlichungstag der Anmeldung:
**15.08.84 Patentblatt 84/33**

(45) Bekanntmachung des Hinweises auf die Patenterteilung:
**24.06.87 Patentblatt 87/26**

(84) Benannte Vertragsstaaten:
**AT DE FR GB IT**

(56) Entgegenhaltungen:
**FR - A - 2 353 275**
**FR - A - 2 450 104**
**US - A - 1 619 569**
**US - A - 4 198 713**

(73) Patentinhaber: **GEBRÜDER SULZER**
**AKTIENGESELLSCHAFT, Zürcherstrasse 9,**
**CH-8401 Winterthur (CH)**

(72) Erfinder: **Frey, Otto, Wallrütlstrasse 56,**
**CH-8400 Winterhur (CH)**

(74) Vertreter: **Dipl.-Ing. H. Marsch Dipl.-Ing. K. Sparing**
**Dipl.-Phys.Dr. W.H. Röhl Patentanwälte,**
**Rethelstrasse 123, D-4000 Düsseldorf (DE)**

ACTORUM AG

## Beschreibung

Die Erfindung betrifft eine Einsteckhülse für den Verankerungsschaft einer zementfrei implantierbaren Kleingelenk-Prothese, welcher Schaft aus einem sich zum freien Ende hin stetig verjüngenden Stiel eckigen Querschnitts besteht, wobei ein Halterungsteil der Hülse den Schaft in seinem gelenknahen Bereich allseitig eng umschliesst.

Kleingelenk-Prothesen, beispielsweise für Finger-, Ellenbogen- oder Handgelenke, sind häufig einteilig ausgebildet, d.h. die Gelenkfunktion wird durch elastische Verformungen eines einzigen, zwei Verankerungsschäfte verbindenden Gelenkteils ausgeübt (DE-B2 2 440 103). Diese Prothesen bestehen daher aus einem relativ weich elastischen Werkstoff, wie z.B. einem Elastomer, das vorwiegend ein Kunststoff – beispielsweise ein Silikonkautschuk, ein Polyolefin oder Polyäthylen – ist. Ihre Verankerungsschäfte sind somit ebenfalls relativ weich und elastisch.

Es ist daher bekannt (FR-A-2 450 104), derartige Gelenkprothesen in ihrem gelenknahen Bereich mit einer Hülse zu umgeben, um die Bruchgefahr für die Prothese und/oder für die sie umgebenden Knochen zu vermindern. Diese bekannten Hülsen, die die Schäfte der Prothesen eng umschliessen, sind ebenfalls aus Kunststoff gefertigt.

Die genannten Kleingelenk-Prothesen aus elastischem Werkstoff führen bei jeder Bewegung des Gelenkes Relativbewegungen gegenüber dem Knochen aus, die beispielsweise beim Abbiegen des Gelenkes aufgrund lokaler Verformungen in dem elastischen Material entstehen und sich als «elastische Wellen» durch dieses hindurch fortpflanzen. Diese sogenannten Mikrobewegungen verursachen besonders auf der «Aussenseite» des Gelenkes, d.h. an der Oberfläche des beim Abbiegen des Gelenkes auf Zug beanspruchten, gestreckten Schaftbereichs, Knochenreizungen, auf die das Knochengewebe mit Abbau reagiert. Derartige Relativbewegungen zwischen Knochen und Implantat werden durch die erwähnte bekannte Hülse nicht verhindert, da beispielsweise die erwähnten «elastischen Wellen» sich in den hülsenfreien Teil des Schaftes fortpflanzen.

Aufgabe der Erfindung ist es, für Verankerungsschäfte der genannten Art eine Halterung zu schaffen, durch die eine Reizung des Knochens durch Bewegungen des Schaftes relativ zum Knochen vermieden werden. Selbstverständlich ist die Erfindung dabei nicht auf die diskutierten, einteiligen Prothesen beschränkt, sondern auch bei mehrteiligen Kleingelenk-Prothesen mit relativ weich-elastischen Schäften anwendbar. Die Lösung der gestellten Aufgabe besteht in einer Einsteckhülse, die gekennzeichnet ist durch eine sich vom Halterungsteil zum freien Ende des Schaftes hin erstreckende Verformungsplatte und daran anschliessend durch ein den Schaft ebenfalls umschliessendes, entlang der Erstreckungsrichtung geschlitztes, am Boden offenes Endstück, ferner ist die Erfindung dadurch gekennzeichnet, dass der Halterungsteil, die Verformungsplatte und das Endstück aus Metall gefertigt sind.

Die Einsteckhülse, die zementfrei im Knochen verankert ist, behält aufgrund der Festigkeit und «Steife» des Metalls ihre Form bei, so dass sich in ihr Bewegungen vom Gelenkbereich her nicht fortpflanzen, sondern ausserhalb des Knochens lokalisiert bleiben. Der gelenknahe Halterungsteil verleiht dem Schaft dabei in bekannter Weise einen festen Sitz in der Hülse, ohne dass Relativbewegungen zwischen beiden, beispielsweise bei Zugbelastungen, ausgeschlossen werden. Vor allem behindert der Halterungsteil die Fortpflanzung der erwähnten «elastischen Wellen» des Schaftes nicht, die bei Bewegungen des Gelenkes unvermeidbar entstehen. Die dabei auftretenden Mikrobewegungen erfolgen nunmehr jedoch gegenüber der Hülse, so dass Knochenreizungen dadurch nicht mehr verursacht werden. Die Verformungsplatte, die sich vorzugsweise nur über eine Seite des eckigen Schaftquerschnittes bzw. auf der besonders belasteten «Aussenseite» des Gelenkes über eine relativ kurze Länge erstreckt, bildet einen relativ leicht verformbaren Abschnitt der Hülse, indem diese durch Verbiegen an die anatomischen Gegebenheiten angepasst werden kann. Das Endstück schliesslich trennt das Knochengewebe vom verbleibenden Teil des Schaftes bis zu dessem freien Ende hin; es ist längs seines Mantels geschlitzt und am Boden offen, um bei Relativbewegungen zwischen Schaft und Hülse kolbenpumpenartige Wirkungen zu verhindern, durch die bei axialen Bewegungen der Prothese Körperflüssigkeit in die Einsteckhülse hineingesogen wird, ohne dass sie wieder abfliessen kann; weiterhin verursacht sich bewegende Flüssigkeit ebenfalls Knochenreizungen, die zu Knochenresorptionen führen.

An ihrem gelenknahen Ende kann die Einsteckhülse bzw. ihr Halterungsteil vorteilhafterweise in abgerundeten Bögen enden, um Beschädigungen des weich-elastischen Gelenkteils und/oder des Gewebes am Rand der Hülse zu vermeiden. Gleichzeitig bilden diese Bögen eine Abstützung der Einsteckhülse auf dem kortikalen Gewebe des sie aufnehmenden Knochens, wodurch ein unzulässig tiefes Eindringen der Hülse in den Knochen verhindert wird.

Im Folgenden wird die Erfindung anhand eines Ausführungsbeispiels im Zusammenhang mit der Zeichnung näher erläutert.

Fig. 1 zeigt in einer Ansicht senkrecht zur Längsachse die neue Einsteckhülse;
Fig. 2 ist der Schnitt II-II und
Fig. 3 derjenige III-III von Fig. 1;
Fig. 4 gibt in einer Ansicht ähnlich derjenigen von Fig. 1 ein durch eine Prothese ersetztes Fingergrundgelenk wieder, wobei die Prothese über je eine Einsteckhülse in die Knochen eingesetzt ist.

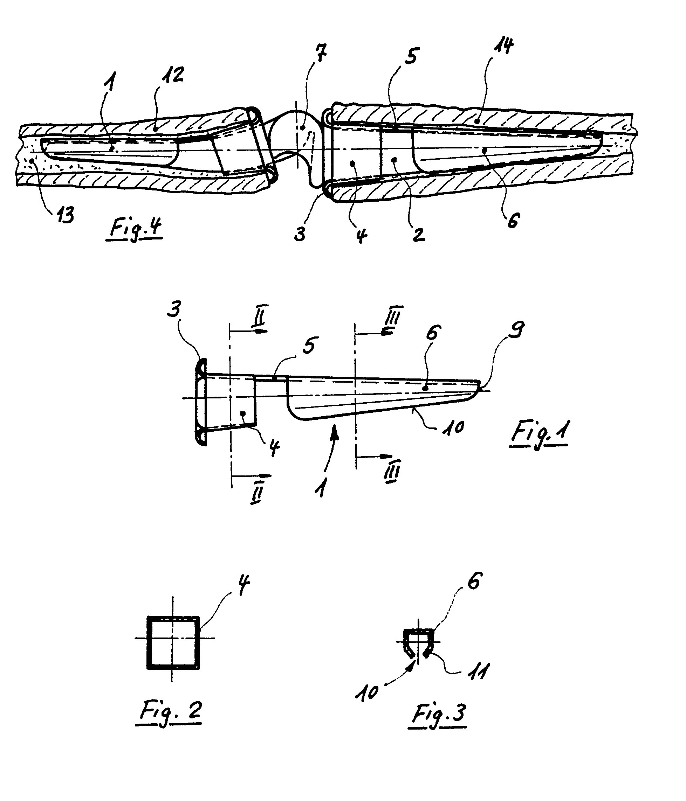

Die Einsteckhülse 1 (Fig. 1) ist entsprechend ihrem Verwendungszweck in ihrer Grundform an den von ihr zu umhüllenden Prothesenschaft 2 (Fig. 4) angepasst. Sie hat daher im vorliegenden Beispiel die Form einer sich zum distalen Ende verjüngenden, hohlen Pyramide mit quadratischem Querschnitt (Fig. 2). Der gelenknahe Rand 3 der aus dünnem Blech von beispielsweise 0,3 mm Dicke hergestellten Einsteckhülse 1, das aus einem der in der Implantat-Technik üblichen Metalle bzw. einer Metall-Legierung besteht, ist zur Vermeidung von Beschädigungen des umgebenden Gewebes bzw. des elastischen Gelenkteils 7 (Fig. 4) der Prothese durch Biegen zu einem nach aussen konvexen Bogen verformt.

An den Rand 3 schliesst sich zunächst der den Schaft 2 relativ eng umschliessende Halterungsteil 4 an, der eine in Umfangsrichtung geschlossene Büchse bildet; das Schliessen des Halterungsteils 4 kann dabei beispielsweise durch Nieten, Schweissen oder Falzen erfolgen. Über eine kurze Verformungsplatte 5 ist der Halterungsteil 4 mit einem Endstück 6 verbunden, das den verbleibenden Teil des Schaftes bis zu seinem freien Ende hin umhüllt; die Verformungsplatte 5 erstreckt sich nur über eine Seite des Querschnitts-Quadrats, und zwar nur über diejenige, die beim Beugen die «Aussenseite» des «gespannten» Gelenks bildet. Die Platte 5 hat vor allem die Aufgabe, eine Anpassung der Einsteckhülse an den sie aufnehmenden Knochenhohlraum zu erleichtern.

Das Endstück 6, das den Rest des Schaftes 2 vom Knochen 14 (Fig. 4) trennt, muss – ähnlich wie die Verformungsplatte 5 – vor allem die «Aussenseite» des Schaftes 2 und die daran anliegenden Seitenflanken abdecken. Es ist daher sowohl an seinem gelenkfernen Ende 9 als auch an seiner – vom Gelenk her gesehen – «Innenseite» 10 offen, wobei diese zu der «Innenseite» gehörige Wand 11 geschlitzt und leicht aufgebogen ist (Fig. 3).

Bei dem prothetisch ersetzten Fingergrundgelenk nach Fig. 4 ist links ein Fingerknochen 12 gezeigt, dessen spongioses Gewebe 13 teilweise ausgeräumt ist. In den entstandenen Hohlraum ist eine Einsteckhülse 1 eingesetzt, die durch Verbiegung der Verformungsplatte 5 an den Verlauf des Knochenhohlraums angepasst worden ist. In der Hülse 1 steckt der Schaft 2 einer einteiligen Prothese, dessen weich-elastischer Gelenkteil 7 das Fingergrundgelenk ersetzt.

Der Gelenkteil 7 geht nach rechts wieder in einen Schaft 2 über, der in einer gleichartigen Einsteckhülse 1 versenkt ist; diese sitzt im spongiosen Gewebe 13 eines Mittelhandknochens 14.

## Patentansprüche

1. Einsteckhülse für den Verankerungsschaft (2) einer zementfrei implantierbaren Kleingelenk-Prothese, welcher Schaft (2) aus einem sich zum freien Ende hin stetig verjüngenden Stiel eckigen Querschnitts besteht, wobei ein Halterungsteil (4) der Hülse den Schaft (2) in seinem gelenknahen Bereich allseitig eng umschliesst, gekennzeichnet durch eine sich vom Halterungsteil (4) zum freien Ende des Schaftes (2) hin erstreckende Verformungsplatte (5), und daran anschliessend durch ein den Schaft (2) ebenfalls umschliessendes, entlang der Erstreckungsrichtung geschlitztes, am Boden offenes Endstück (6), wobei der Halterungsteil (4), die Verformungsplatte (5) und das Endstück (6) aus Metall gefertigt sind.

2. Einsteckhülse nach Anspruch 1, dadurch gekennzeichnet, dass der Halterungsteil (4) längsseitig in nach aussen abgerundeten Bögen (3) endet.

## Claims

1. An insert sleeve for the anchor stem (2) of a cementlessly implantable small articulation prosthesis, the stem (2) being in the form of a polygonal-section rod or stick or the like which narrows continuously towards its free end, a retaining part (4) of the sleeve extending closely all round the stem (2) in that part thereof which is near the joint, characterised by a deformation plate (5) which extends from the retaining part (4) towards the free end of the stem (2) and which is followed by an end member (6) which also extends around the stem (2), is slotted along the direction in which it extends and is open on the base, the retaining part (4), deformation plate (5) and end member (6) being made of metal.

2. An insert sleeve according to claim 1, characterised in that the retaining part (4) terminates longitudinally in outwardly rounded curves (3).

## Revendications

1. Douille emboîtable pour la tige d'ancrage (2) d'une prothèse pour petites articulations implantable sans ciment, laquelle tige (2) est constituée par une queue de section polygonale à rétrécissement continu vers l'extrémité libre, une partie de retenue (4) de la douille entourant étroitement la tige (2) de toutes parts dans sa région proche de l'articulation, caractérisée par une plaquette déformable (5) qui s'étend de la partie de retenue (4) vers l'extrémité libre de la tige (2) et, en juxtaposition, par une pièce d'extrémité (6) à fond ouvert qui entoure également la tige (2) et est fendue le long de la direction de son étendue, la partie de retenue (4), la plaquette déformable (5) et la pièce d'extrémité (6) étant fabriquées en métal.

2. Douille emboîtable selon la revendication 1, caractérisée par le fait que la partie de retenue (4) s'achève, du côté longitudinal, par des arceaux (3) arrondis vers l'extérieur.

Fig.4

Fig.1

Fig. 2

Fig.3